# EUROPÄISCHE PATENTANMELDUNG

(11) **EP 2 717 051 A2**
(43) Veröffentlichungstag der Anmeldung: **09.04.2014**
(21) Anmeldenummer: 13186697.2
(22) Anmeldetag: 30.09.2013
(51) Int. Cl.: G01N 33/00

(54) **Flüssigkeitsrückhaltevorrichtung**

(30) Priorität: 08.10.2012 DE 202012103850 U
(71) Anmelder: IT Inventor GmbH, 48465 Samern (DE)
(72) Erfinder: Thannhäuser, Ingo, 48465 Samern (DE)
(74) Vertreter: Werner & ten Brink

(57) **Zusammenfassung**

Die Erfindung ist eine Flüssigkeitsrückhaltevorrichtung (10) mit einem Sensorteil (12) und einem mittels des Sensorteils (12) ansteuerbaren Aktorteil (14), wobei das Sensorteil (12) eine in Form zumindest eines Glykolsensors (30, 32) realisierte Sensorik (30, 32) in einem einseitig offenen Gehäuse (34) umfasst und wobei die Sensorik (30, 32) in dem Gehäuse (34) durch eine wasserundurchlässige, aber dampfdiffusionsoffene Membran (38, 40) von einer Gehäuseöffnung (36) zurückgesetzt ist.

## Beschreibung

Die Erfindung betrifft eine Flüssigkeitsrückhaltevorrichtung, die zur Verwendung mit einer Auffangwanne unter einem leckagegefährdeten Aggregat mit einem darin befindlichen wassergefährdenden, flüssigen und zurückzuhaltendem Medium sowie zur Ansteuerung eines der Auffangwanne, nämlich einem Abfluss oder Überlauf der Auffangwanne, zugeordneten Ventils bestimmt und eingerichtet ist.

Die hier und im Folgenden beschriebene Flüssigkeitsrückhaltevorrichtung kommt auch zur Verwendung mit einem Abscheider in Betracht, wie er zum Beispiel aus der EP 2 335 794 B bekannt ist. In der EP 2 335 794 B ist die dort beschriebene Vorrichtung ebenfalls als Flüssigkeitsrückhaltevorrichtung bezeichnet, so dass sie zur Unterscheidung im Weiteren als Abscheider bezeichnet wird. Der Abscheider zielt auf das Abscheiden und Zurückhalten von nicht wasserlöslichen Stoffen ab. Die im Folgenden beschriebene Flüssigkeitsrückhaltevorrichtung zielt auf das Zurückhalten auch von wasserlöslichen Stoffen ab. Eine Kombination des Abscheiders aus der EP 2 335 794 B oder eines ähnlichen Abscheiders und der im Folgenden beschriebenen Flüssigkeitsrückhaltevorrichtung ermöglicht das Zurückhalten von nicht wasserlöslichen und von wasserlöslichen Stoffen. Ein Beispiel für solche wasserlöslichen Stoffe ist Glykol.

Glykol (umgangssprachliche Bezeichnung für (Mono-) Ethylenglykol) wird bekanntlich als Frostschutzmittel, zum Beispiel in Klima- oder Kälteanlagen, eingesetzt. Im Falle einer Leckage solcher Anlagen droht ein Austritt des Frostschutzmittels. Weil Glykol als wassergefährdender Stoff eingestuft und überdies gesundheitsschädlich ist, muss bei einer Leckage speziell ein Versickern des Frostschutzmittels oder ein sonstiger Übergang des Frostschutzmittels in die Umwelt verhindert werden.

Weil Glykol sehr gut wasserlöslich ist, lässt sich Glykol - anders als zum Beispiel hydrophobe Stoffe wie Öl und dergleichen - mit einem Flüssigkeitsabscheider oder einer Rückhaltevorrichtung, die auf einer Phasenbildung der abzuscheidenden/ zurückzuhaltenden Flüssigkeit einerseits und Wasser andererseits basieren, nicht abscheiden/zurückhalten. Die bisherigen Lösungen zur Vermeidung eines Übertritts von Glykol in die Umwelt basieren daher auf der Verwendung von Auffangwannen, die unter den jeweiligen leckagegefährdeten Anlagen aufgebaut werden oder in denen die jeweiligen leckagegefährdeten Anlagen platziert werden. Eine Auffangwanne ist dabei bisher stets so dimensioniert, dass eine bestimmte Menge Regenwasser zusätzlich zu eventuell ausgetretenem Glykol aufgenommen werden kann, ohne dass es zu einem Überlauf kommt. Die Dimensionierung einer Auffangwanne ergibt sich dabei speziell aus am Aufstellungsort in der Vergangenheit beobachteten maximalen Regenmengen einerseits und einem realistischen Wartungs-/Prüfzyklus andererseits. Bei einer Leckage sind eine Entleerung der Auffangwanne und eine anschließende sachgerechte Entsorgung des in der Auffangwanne aufgefangenen Glykol-Wasser-Gemisches erforderlich. Zur Vermeidung starrer Wartungs-/Prüfzyklen, die eine optische Inspektion der Auffangwanne(n) durch entsprechend geschultes Personal erfordern, kann eine Drucküberwachung der jeweiligen Anlage vorgesehen sein, wobei ein Abfallen eines in der jeweiligen Anlage gemessenen Druckmesswerts unter einen vorgegebenen Grenzwert auf eine Leckage der Anlage hindeutet und daraus ein Alarmsignal für Wartungspersonal generiert wird, das daraufhin die Auffangwanne(n) prüft und gegebenenfalls für eine Entsorgung eines dort aufgefangenen Glykol-Wasser-Gemisches sorgt.

Ein solches Vorgehen ist aufwändig und zudem fehleranfällig, wenn nämlich das Alarmsignal nicht beachtet oder nicht wahrgenommen wird oder wenn das Alarmsignal zusammen mit anderen Alarmsignalen eintrifft und das Wartungspersonal entsprechend eine Entscheidung treffen muss, welcher Alarmmeldung zunächst nachgegangen wird, oder das Wartungspersonal sogar aufgrund einer übergeordneten Alarmmeldung veranlasst wird, ein jeweiliges Betriebsgelände zu verlassen.

Eine Aufgabe der vorliegenden Erfindung besteht entsprechend darin, eine Flüssigkeitsrückhaltevorrichtung für wasserlösliche Stoffe anzugeben, die die oben skizzierten Nachteile vermeidet oder zumindest reduziert.

Diese Aufgabe wird erfindungsgemäß mit einer Flüssigkeitsrückhaltevorrichtung der eingangs genannten Art mit den Merkmalen des Anspruchs 1 gelöst. Dazu ist bei einer Flüssigkeitsrückhaltevorrichtung mit einem Sensorteil und einem Aktorteil zur Verwendung mit einer Auffangwanne unter einem leckagegefährdeten Aggregat mit einem darin befindlichen wassergefährdenden, flüssigen und zurückzuhaltendem Medium Folgendes vorgesehen: Mittels einer von dem Sensorteil umfassten Sensorik ist das wassergefährdende Medium in der Auffangwanne sensierbar und mittels des Sensorteils ist gegebenenfalls ein diesbezügliches Sensorsignal generierbar. Das Aktorteil ist mittels des Sensorsignals, nämlich mittels des von dem Sensorteil generierten Sensorsignals, ansteuerbar. Das Aktorteil fungiert als Sicherheitseinrichtung oder umfasst eine Sicherheitseinrichtung, mittels derer ein Austritt des wassergefährdenden flüssigen Mediums aus der Auffangwanne verhinderbar ist. Als Sensorik des Sensorteils fungiert zumindest ein Glykolsensor.

Im Interesse einer besseren Lesbarkeit der Beschreibung wird im Folgenden die Sensorik des Sensorteils nicht stets als "der mindestens eine Glykolsensor" oder "der oder jeder Glykolsensor" oder dergleichen, sondern nur kurz als Sensorik bezeichnet. Dabei ist allerdings stets mitzulesen, dass die Sensorik genau einen oder mehr als einen Glykolsensor umfassen kann.

Im Zusammenhang mit der Beschreibung des Abscheiders in der eingangs erwähnten EP 2 335 794 B wird darauf hingewiesen, dass dem Abscheider ein Ventil zugeordnet sein kann, dessen Ansteuerung mittels einer Sensorik erfolgt, welche entweder einen Druckabfall in einem leckagegefährdeten Aggregat oder mittels einer Leitfähigkeitsmessung eine Verunreinigung der Flüssigkeit in einer den Abscheider umgebenden Auffangwanne erkennt.

Ein Sensor zur Messung einer Alkoholkonzentration in einer wässrigen Lösung ist aus der EP 0 867 713 A2, der DE 103 02 220 A1 und der AT 502 948 A2 bekannt. Die hier vorgeschlagene Flüssigkeitsrückhaltevorrichtung umfasst eine Auffangwanne der oben skizzierten Art oder ist zur Kombination mit einer solchen Auffangwanne bestimmt. Wenn die Auffangwanne nach oben offen ist, kann diese in einfacher Art und Weise unter einer Kälte- oder Klimaanlage oder einem Aggregat, welches im Fehlerfall Glykol oder ein Glykolgemisch abgibt, so dass Glykol oder Glykolgemisch in die Auffangwanne abläuft oder tropft, positioniert werden. Die Flüssigkeitsrückhaltevorrichtung ist dann mit ihrem Sensorteil in der Lage, den Inhalt der Auffangwanne auf eine eventuelle Anwesenheit von Glykol zu überwachen und durch Ansteuerung des Aktorteils der Flüssigkeitsrückhaltevorrichtung wird ein Ausfluss von Glykol oder einem Glykolgemisch aus der Auffangwanne sicher verhindert.

Im Folgenden werden für eine einfache und besser lesbare Beschreibung sämtliche Anlagen oder Aggregate, die im Fehlerfall Glykol oder dergleichen abgeben, ohne Verzicht auf eine weitergehende Allgemeingültigkeit als Klimaanlage bzw. Klimaanlagen bezeichnet. Jede Art von Auffangwanne oder Auffangbehälter, die oder der ganz oder teilweise unter einer solchen Anlage platzierbar ist und zum Auffangen von Glykol oder dergleichen bestimmt oder geeignet ist, wird im Folgenden als Auffangwanne bezeichnet. Dabei ist der Begriff "unter" nicht notwendig räumlich zu verstehen und meint vielmehr auch eine Anordnung in Richtung einer sich aufgrund von Gravitation ergebenden Flussrichtung des jeweiligen Mediums, etwa wenn sich unterhalb der Klimaanlage ein Leitblech oder dergleichen befindet, das austretende Flüssigkeit unterhalb der Klimaanlage in einen Bereich unterhalb, aber neben der Klimaanlage und in eine dortige Auffangwanne leitet. Gleiches gilt für Sonnenkollektoren und dergleichen, die ebenfalls ein Beispiel für Aggregate sind, die im Fehlerfall Glykol oder Ähnliches abgeben, und oftmals auf geneigten Dachflächen angebracht sind. Dann kann als Auffangwanne sogar eine Dachrinne fungieren. Des Weiteren wird die nachfolgende Beschreibung - ebenfalls ohne Verzicht auf eine weitergehende Allgemeingültigkeit und entsprechend nur beispielhaft - unter Bezugnahme auf Glykol als aus der Klimaanlage eventuell austretendem wassergefährdenden Stoff fortgesetzt.

Der Vorteil der Erfindung besteht darin, dass mit dem Sensorteil der Flüssigkeitsrückhaltevorrichtung eine einfache und kontinuierliche Überwachung einer jeweiligen Auffangwanne oder dergleichen auf eine eventuelle Anwesenheit von Glykol möglich ist. Das Sensorteil wird im Folgenden entsprechend mitunter auch als Glykolsensorvorrichtung bezeichnet. Die Überwachung mittels des Sensorteils ist sehr sicher, weil im Fehlerfall direkt die Anwesenheit von Glykol und dies auch in geringsten Mengen sensiert werden kann. Damit kann bei einer solchen Erkennung von Glykol unmittelbar und automatisch mittels des Aktorteils der Flüssigkeitsrückhaltevorrichtung eine Verriegelung eines Abflusses der Auffangwanne veranlasst werden, zum Beispiel durch eine entsprechende Ansteuerung eines der Auffangwanne zugeordneten und als Aktorteil fungierenden Ventils.

Die Reaktionszeit von der Sensierung des Glykols bis zur Ansteuerung des Aktorteils, also zum Beispiel bis zum Schließen eines jeweiligen Ventils, ist dabei systembedingt deutlich günstiger als dies bei einem bisher im Stand der Technik verwendeten Wirkpfad mit einer Drucküberwachung oder einer Leitfähigkeitsmessung der Fall sein kann. Eine Drucküberwachung der Kälte- oder Klimaanlage muss stets betriebs- oder umgebungsbedingte Druckschwankungen berücksichtigen, zum Beispiel Druckschwankungen, die sich aufgrund von tageszeit- oder jahreszeitbedingten Temperaturschwankungen ergeben. Dies bedeutet, dass eine Drucküberwachung nicht auf beliebige Druckänderungen, sondern erst bei einer erheblichen Druckänderung reagieren darf. Bis der für das Ansprechen einer solchen Drucküberwachung vorgegebene Grenzwert erreicht ist, tritt aber kontinuierlich Frostschutzmittel und damit Glykol oder Ähnliches aus und kann gegebenenfalls bereits aus der Auffangwanne abfließen und in die Umwelt gelangen. Gleiches gilt sinngemäß im Falle einer Leitfähigkeitsmessung, denn die Leitfähigkeit eines Stoffs hängt bekanntlich ebenfalls von Umwelteinflüssen, hier speziell der Umgebungstemperatur, ab. Mittels des Sensorteils wird die Anwesenheit auch geringer Glykolmengen in der Auffangwanne sensiert und die Auffangwanne kann unmittelbar durch entsprechende Ansteuerung eines Ventils oder dergleichen verriegelt werden. Bei einer Anbringung des Sensorteils vor (stromaufwärts der Flussrichtung durch den Auslass) einem Auslass aus der Auffangwanne ist normalerweise, vor allem bei einem Ventil mit einer entsprechend geringen Schließdauer, sichergestellt, dass kein Glykol aus der Auffangwanne austreten kann.

Als Glykolsensor kommt ein elektronisches Bauteil in Betracht, wie es von dem Unternehmen AppliedSensor unter der Bezeichnung iAQ-engine angeboten wird. Dieser Glykolsensor arbeitet als Halbleiter-Gassensor / Diffusionssensor und ist damit in der Lage, gasförmige Alkohole und damit auch Glykol, bei dem es sich bekanntlich um einen zweiwertigen Alkohol handelt, zu sensieren.

Vorteilhafte Ausgestaltungen der Erfindung sind Gegenstand der Unteransprüche. Dabei verwendete Rückbeziehungen weisen auf die weitere Ausbildung des Gegenstands des Hauptanspruches durch die Merkmale des jeweiligen Unteranspruches hin. Sie sind nicht als ein Verzicht auf die Erzielung eines selbständigen, gegenständlichen Schutzes für die Merkmalskombinationen der rückbezogenen Unteransprüche zu verstehen. Des Weiteren ist im Hinblick auf eine Auslegung der Ansprüche bei einer näheren Konkretisierung eines Merkmals in einem nachgeordneten Anspruch davon auszugehen, dass eine derartige Beschränkung in den jeweils vorangehenden Ansprüchen nicht vorhanden ist.

Bei einer besonderen Ausführungsform des Sensorteils der Flüssigkeitsrückhaltevorrichtung umfasst dieses als Sensorik zumindest zwei Glykolsensoren, also zumindest einen ersten und einen zweiten Glykolsensor. Die zumindest zwei Glykolsensoren ermöglichen eine Verwendung des Sensorteils und damit eine Überwachung und Erkennung eines eventuellen Glykolaustritts auch dann noch, wenn einer der Glykolsensoren, zum Beispiel aufgrund eines Defekts oder aufgrund von Verschmutzung, ausfällt, so dass sich eine Redundanz ergibt. Alternativ können die mindestens zwei Glykolsensoren auch zur Plausibilitätsprüfung vor Abgabe eines Signals zur Ansteuerung des Aktorteils der Flüssigkeitsrückhaltevorrichtung verwendet werden. Schließlich können die mindestens zwei Glykolsensoren auch zeitlich versetzt ausgewertet werden, so dass sich eine Verringerung der Reaktionszeit des Sensorteils im Falle eines Glykolaustritts ergibt.

Indem das Sensorteil der Flüssigkeitsrückhaltevorrichtung (Glykolsensorvorrichtung) in einem einseitig offenen Gehäuse angeordnet und in dem Gehäuse durch eine wasserundurchlässige, aber dampf- und/oder gasdiffusionsoffene Membran von der Gehäuseöffnung zurückgesetzt ist, können mittels des Sensorteils und dessen Sensorik sensierbare Gase, also insbesondere über einer Wasseroberfläche mit Verunreinigungen in Form von Glykol oder dergleichen aufsteigende Dämpfe, die dampf- und/oder gasdiffusionsoffene Membran passieren und gelangen so zu der von dem Sensorteil umfassten Sensorik. Bei der wasserundurchlässigen, aber dampf- und/oder gasdiffusionsoffenen Membran - im Folgenden kurz nur als Membran bezeichnet - handelt es sich zum Beispiel um eine Membran, wie sie unter der Marke Goretex allgemein bekannt ist. Andere gleichwertige wasserundurchlässige und dampf- und/oder gasdiffusionsoffene Membranen kommen selbstverständlich ebenso in Betracht. Indem die Membran wasserundurchlässig ist, ist die Sensorik im Inneren des Sensorteils wassergeschützt. Dies betrifft die Sensorik selbst, aber auch deren elektrische Anschlüsse sowie eine Verarbeitungseinrichtung in Form eines Mikroprozessors oder dergleichen zur Ansteuerung und Überwachung der Sensorik. Die aufgrund der genannten Membran wassergeschützte Anbringung der Sensorik und sämtlicher Mittel für deren elektrische Beschaltung erlaubt eine unkomplizierte Anbringung des Sensorteils zum Beispiel über einer Auffangwanne.

Bei einer Ausführungsform des Sensorteils weist dieses ein Schutzgitter vor der Membran auf. Relative räumliche Bezeichnungen wie "vor" oder dergleichen beziehen sich hier und im Folgenden auf die Sensorik als zentrale Funktionseinheit des Sensorteils. Bei einem vor der Membran liegenden Schutzgitter ergibt sich also von der Sensorik aus gesehen folgende Abfolge: Glykolsensor(en), Membran, Schutzgitter. Das Schutzgitter oder jede sonstige geeignete Schutzeinrichtung, zum Beispiel eine Glas- oder Kunststoffscheibe mit einer Vielzahl von Löchern oder Durchbrüchen, schützt die Membran vor eventuellen mechanischen Beschädigungen und schützt damit mittelbar auch die Sensorik im Inneren des Sensorteils.

Bei einer weiteren Ausführungsform des Sensorteils ist vorgesehen, dass dieses einen Feuchtigkeitssensor im Bereich der Sensorik umfasst. Als Anbringungsort für den Feuchtigkeitssensor im Bereich der Sensorik kommt dabei speziell eine Anbringung in Betracht, bei der sich der Feuchtigkeitssensor auf derselben Seite der Membran befindet wie die Sensorik. Der Feuchtigkeitssensor kommt dabei speziell für eine Aktivierung der Sensorik in Betracht, derart, dass die Sensorik nur dann ein Sensorsignal liefert oder ein eventuell kontinuierlich von der Sensorik geliefertes Sensorsignal nur dann verarbeitet wird, wenn der Feuchtigkeitssensor Feuchtigkeit im Bereich des Sensorteils, insbesondere unterhalb des Sensorteils, sensiert. Der Feuchtigkeitssensor oder ein vom Feuchtigkeitssensor geliefertes Feuchtigkeitssensorsignal ermöglicht damit eine Überwachung der Sensorik oder eine Plausibilitätskontrolle eines von der Sensorik gelieferten Sensorsignals. Diese Verwendung des Feuchtigkeitssensors basiert auf dem Umstand, dass jede zum Auffangen von Glykol vorgesehene Auffangwanne so dimensioniert ist, dass die gesamte potentiell austretende Glykolmenge oder ein gesamtes potentiell austretendes Glykol-Wasser-Gemisch von der Auffangwanne aufgenommen werden kann, ohne dass es zu einem Austritt von Glykol aus der Auffangwanne kommt. Zu einem Austritt kann es demnach erst kommen, wenn in die Auffangwanne weitere Flüssigkeit, zum Beispiel in Form von Regenwasser, gelangt. Eine eventuelle Verriegelung eines Abflusses der Auffangwanne ist damit normalerweise erst erforderlich, wenn Regenwasser in die Auffangwanne gelangt. Die Anwesenheit von Regenwasser in der Auffangwanne kann mit dem Feuchtigkeitssensor erkannt werden und eine Verknüpfung eines vom Feuchtigkeitssensor gelieferten Signals mit einem von der Sensorik gelieferten Signal dazu verwendet werden, um gegebenenfalls die Auffangwanne durch Ansteuerung des Aktorteils der Flüssigkeitsrückhaltevorrichtung zu verschließen.

Bei einer Ausführungsform der Flüssigkeitsrückhaltevorrichtung weist diese, insbesondere als Bestandteil des Sensorteils, eine Verarbeitungseinheit und einen Speicher, gegebenenfalls eine einen Speicher umfassende Verarbeitungseinheit, jeweils in Form von oder nach Art eines Mikroprozessors auf, wobei in dem Speicher ein durch die Verarbeitungseinheit ausführbares Steuerungsprogramm gespeichert ist, das im Betrieb der Flüssigkeitsrückhaltevorrichtung die Sensorik des Sensorteils ansteuert und überwacht. In dem Speicher ist ein durch die Verarbeitungseinheit mit einem von der Sensorik gelieferten Messwert vergleichbarer Grenzwert gespeichert, so dass mittels der Verarbeitungseinheit im Falle einer Überschreitung des Grenzwerts durch den Messwert ein Sensorsignal zur Ansteuerung des Aktorteils der Flüssigkeitsrückhaltevorrichtung generierbar ist. Die Verarbeitungseinheit kann auch in Form eines anwenderspezifischen integrierten Schaltkreises (ASIC) oder dergleichen realisiert und das Steuerungsprogramm entsprechend in Firmware implementiert sein. Jedenfalls wird durch die Verarbeitungseinheit ein von der Sensorik gelieferter Messwert mit dem Grenzwert verglichen und bei Überschreiten des Grenzwerts ein Sensorsignal generiert. Grundsätzlich ist auch eine Ausführungsform der Flüssigkeitsrückhaltevorrichtung denkbar, bei welcher das Sensorteil kontinuierlich ein dem jeweiligen Messwert entsprechendes Signal liefert und der Vergleich mit dem Grenzwert außerhalb des Sensorteils, also zum Beispiel im Aktorteil oder einer in Signalflussrichtung zwischen Sensor- und Aktorteil angeordneten Funktionseinheit, erfolgt.

Bei einer besonderen Ausführungsform der Flüssigkeitsrückhaltevorrichtung und einer Auswertung eines von der Sensorik des Sensorteils gelieferten Messwerts anhand eines Grenzwerts ist vorgesehen, dass das Sensorsignal dann generierbar ist und im Betrieb der Flüssigkeitsrückhaltevorrichtung dann generiert wird, wenn der Messwert einen unterhalb des Grenzwerts liegenden Schwellwert überschreitet und im Anschluss an das Überschreiten des Schwellwerts innerhalb einer vorgegebenen oder vorgebbaren Zeitspanne ein Anstieg des Messwerts um einen vorgegebenen oder vorgebbaren Betrag sensiert wird. Eine solche Auswertung des Messwerts hilft, Fehlauswertungen zu vermeiden. Kurz gefasst ist bei einer solchen Auswertung für die Generierung des Sensorsignals eine Situation erforderlich, bei der zumindest einmalig ein Messwert sensiert wird, der oberhalb des Schwellwerts liegt und bei dem im Anschluss daran der Messwert ansteigt. Die Art des für die Generierung des Sensorsignals erforderlichen Anstiegs wird dabei durch die jeweils spezifizierte Dauer und den jeweils spezifizierten Betrag vorgegeben. Wenn innerhalb der vorgegebenen Dauer kein Anstieg des Messwerts um den vorgegebenen Betrag erfolgt, wird das anfängliche Überschreiten des Schwellwerts durch den von der Sensorik erhaltenen Messwert als Sensorfehler interpretiert und die Auswertung des Messwerts beginnt erneut mit einer Überwachung des Messwerts in Bezug auf ein eventuelles Überschreiten des Grenzwerts oder ein eventuelles Überschreiten des Schwellwerts.

Wenn das Sensorteil eine Busschnittstelle, zum Beispiel eine Busschnittstelle für einen Anschluss an den sogenannten CAN-Bus, sowie eine über einen an die Busschnittstelle angeschlossenen Bus parametrierbare Verarbeitungseinheit und eine Verarbeitungseinheit zur Generierung eines über einen an die Busschnittstelle angeschlossenen Bus weiterleitbaren Sensorsignals aufweist, kann das Sensorteil mit anderen busfähigen Geräten, insbesondere anderen Sensorteilen, an einem Bus betrieben werden. Die Anschlussmöglichkeit an einen Bus macht die Verwendung des Sensorteils besonders einfach, weil für einen elektrischen Anschluss die Busverbindung ausreicht und eine sonst über eine direkte elektrische Verdrahtung erfolgende Zuordnung zwischen einem zunächst unspezifischen elektrischen Signal und einem Entstehungsort des Signals, nämlich zum Beispiel genau einem Sensorteil innerhalb einer Gruppe mehrerer Sensorteile, flexibel im Rahmen einer projektierbaren Auswertung der über den Bus übermittelten Signale und einer entsprechenden, an sich bekannten Adressierung der einzelnen Kommunikationsteilnehmer, also des oder jedes Sensorteils, festgelegt werden kann. Mit an einen Bus anschließbaren Sensorteilen kann somit in besonders einfacher Art an einer zentralen Stelle, zum Beispiel einem Anlagenleitstand oder dergleichen, ein Glykolaustritt angezeigt werden, wobei die Anzeige entweder eine Bezeichnung des jeweiligen Sensorteils oder eine räumliche Darstellung des Anbringungsorts des Sensorteils umfasst, so dass für Bedienpersonal das Auffinden des jeweiligen Sensorteils und damit des jeweiligen Fehlerorts besonders einfach ist.

Schließlich betrifft die Erfindung auch ein als Glykolsensorvorrichtung fungierendes Sensorteil der hier und im Folgenden beschriebenen Art. Ein solches Sensorteil ist in einer gegenüber einem Bodenniveau der Auffangwanne erhöhten Position angebracht. Die erhöhte Position gewährleistet, dass das Sensorteil normalerweise nicht direkt mit einer in der Auffangwanne befindlichen Flüssigkeit in Kontakt kommt, so dass auch unabhängig von der in dem Sensorteil normalerweise zum Schutz von dessen Sensorik vorhandenen Membran ein reibungsloser Betrieb des Sensorteils und eine kontinuierliche Überwachung des Inhalts der Auffangwanne auf eine eventuelle Anwesenheit von Glykol möglich ist. Aus der Auffangwanne aufsteigende Dämpfe gelangen dabei zum Sensorteil und dessen Sensorik und führen im Falle von Glykoldämpfen - oder allgemein alkoholischen Dämpfen - zu einer Auslösung des Sensorsignals.

Der Vorteil der Erfindung und ihrer Ausgestaltungen besteht insbesondere auch in der universellen Einsetzbarkeit des Sensorteils. Neben den bisher beschriebenen Anwendungsfällen im Zusammenhang mit einer Auffangwanne ist nämlich zum Beispiel auch eine Überwachung von Solaranlagen oder dergleichen möglich. Bei Solaranlagen mit Sonnenkollektoren wird bekanntlich ebenfalls oftmals Glykol oder Ähnliches als Kältemittel verwendet, das sich bei Sonneneinstrahlung erwärmt und in einem Wärmetauscher die aufgenommene Wärme für Heizzwecke oder zur Gewinnung elektrischer Energie abgibt. Im Falle einer Leckage eines Sonnenkollektors oder der die Sonnenkollektoren verbindenden Verrohrung tritt das Kältemittel in die Umgebung, also häufig zum Beispiel in eine Dachrinne oder dergleichen, aus und kann von dort in die Umwelt gelangen. Das hier beschriebene Sensorteil kommt für solche Situationen auch zur Platzierung in einer Dachrinne oder in einem Fallrohr in Betracht und wo oben von einer Ansteuerung eines der Auffangwanne zugeordneten Ventils gesprochen wurde, würde das Sensorteil hier zum Beispiel eine Ansteuerung einer Ventilklappe im einem Fallrohr bewirken und auf diese Weise verhindern, dass Glykol in die Umwelt gelangt. Weitere Anwendungsfälle, die von der mit dem Sensorteil und deren Sensorik möglichen direkten Glykolmessung und der damit realisierbaren Reaktionszeit auf eine Glykolerkennung profitieren, sind selbstverständlich ebenfalls denkbar.

Die mit der Anmeldung eingereichten Ansprüche sind Formulierungsvorschläge ohne Präjudiz für die Erzielung weitergehenden Schutzes. Da speziell die Gegenstände der Unteransprüche im Hinblick auf den Stand der Technik am Prioritätstag eigene und unabhängige Erfindungen bilden können, behält die Anmelderin sich vor, diese oder noch weitere, bisher nur in der Beschreibung und/oder Zeichnung offenbarte Merkmalskombinationen zum Gegenstand unabhängiger Ansprüche oder Teilungserklärungen zu machen. Sie können weiterhin auch selbständige Erfindungen enthalten, die eine von den Gegenständen der vorhergehenden Unteransprüche unabhängige Gestaltung aufweisen.

Nachfolgend wird ein Ausführungsbeispiel der Erfindung anhand der Zeichnung näher erläutert. Einander entsprechende Gegenstände oder Elemente sind in allen Figuren mit den gleichen Bezugszeichen versehen. Das oder jedes Ausführungsbeispiel ist nicht als Einschränkung der Erfindung zu verstehen. Vielmehr sind im Rahmen der vorliegenden Offenbarung auch Abänderungen und Modifikationen möglich, insbesondere solche Varianten, die zum Beispiel durch Kombination oder Abwandlung von einzelnen in Verbindung mit den im allgemeinen oder speziellen Beschreibungsteil beschriebenen sowie in den Ansprüchen und/oder der Zeichnung enthaltenen Merkmalen bzw. Elementen oder Verfahrensschritten für den Fachmann im Hinblick auf die Lösung der Aufgabe entnehmbar sind und durch kombinierbare Merkmale zu einem neuen Gegenstand oder zu neuen Verfahrensschritten bzw. Verfahrensschrittfolgen führen.

Es zeigen
- Fig. 1: eine Flüssigkeitsrückhaltevorrichtung mit einem davon umfassten Sensorteil und einem davon ebenfalls umfassten Aktorteil,
- Fig. 2: einen Schnitt durch eine Ausführungsform des Sensorteils aus Fig. 1,
- Fig. 3: einen Schnitt durch eine alternative Ausführungsform des Sensorteils aus Fig. 1.

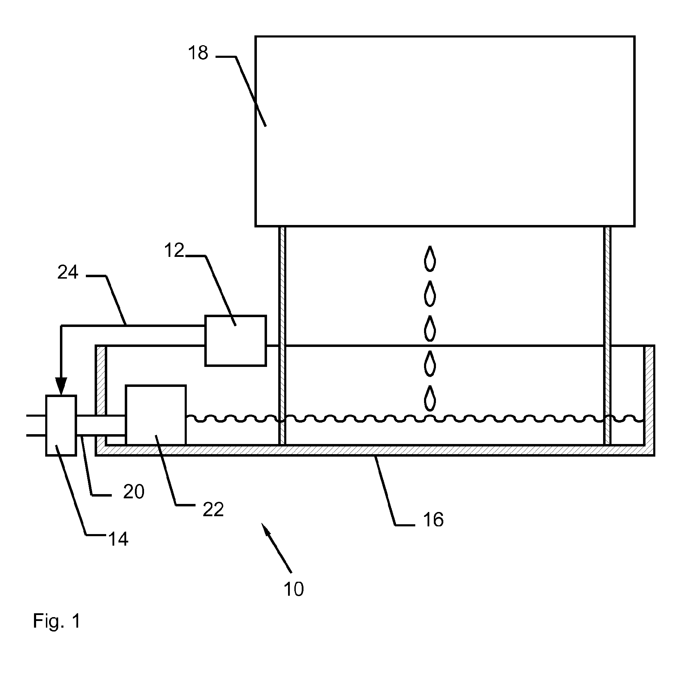

Die Darstellung in Figur 1 zeigt schematisch vereinfacht in einer teilweise geschnittenen Seitenansicht ein Ausführungsbeispiel einer erfindungsgemäßen Flüssigkeitsrückhaltevorrichtung 10. Diese umfasst ein Sensorteil 12 und ein Aktorteil 14 und ist in einer Auffangwanne 16 angeordnet oder der Auffangwanne 16 zugeordnet, so dass die Auffangwanne 16 grundsätzlich ebenfalls als der Flüssigkeitsrückhaltevorrichtung 10 zugehörig aufgefasst werden kann. Die Auffangwanne 16 befindet sich unter einer Klimaanlage 18 oder dergleichen, die hier als Beispiel für ein Aggregat genannt ist, aus dem im Falle einer Leckage ein flüssiges, wassergefährdendes Medium, zum Beispiel Glykol, austritt, wie dies in der Darstellung in Figur 1 durch die Tropfenlinie angedeutet ist.

Im Falle einer solchen Leckage wird das aus der Klimaanlage 18 austretende flüssige Medium von der Auffangwanne 16 aufgefangen und ein aufgrund des austretenden Mediums und/oder gegebenenfalls in sonstiger Weise zuströmender Flüssigkeit, zum Beispiel Regenwasser, resultierender Flüssigkeitsspiegel in der Auffangwanne 16 ist in der Darstellung in Figur 1 symbolisch in Form einer Wellenlinie eingezeichnet. Es besteht allerdings die Gefahr, dass das Glykol über einen Abfluss 20 oder einen Überlauf oder dergleichen aus der Auffangwanne 16 austritt und so ins Erdreich oder in umliegende Gewässer gelangt. Diese Gefahr besteht auch dann, wenn zusammen mit der Auffangwanne 16 ein Abscheider 22, wie er zum Beispiel in der EP 2 335 794 B beschrieben ist, verwendet wird, weil ein solcher Abscheider 22 nur nicht-wasserlösliche Stoffe, also zum Beispiel kein Glykol, abscheiden und zurückhalten kann.

Die hier vorgeschlagene Flüssigkeitsrückhaltevorrichtung 10 sensiert (erkennt) mittels des von der Flüssigkeitsrückhaltevorrichtung 10 umfassten Sensorteils 12 die Anwesenheit von Glykol in der Auffangwanne 16 und steuert mittels eines Sensorsignals 24 gegebenenfalls das von der Flüssigkeitsrückhaltevorrichtung 10 umfasste Aktorteil 14 an, welches einen Abfluss 20, einen Überlauf oder dergleichen der Auffangwanne 16 verschließt. Das Aktorteil 14 ist also zum Beispiel ein Ventil oder umfasst ein Ventil. Auf diese Weise wird der Austritt von Glykol oder dem jeweils sensierten wassergefährdenden Stoff aus der Auffangwanne 16 sicher verhindert. Das Aktorteil 14 kann sich im Inneren der Auffangwanne 16 oder - wie dargestellt - außerhalb der Auffangwanne 16 befinden. Das Sensorteil 12 und/oder das Aktorteil 14 kann bzw. können sich zum Beispiel auch im Inneren des Abscheiders 22 befinden. Des Weiteren können Sensor- und Aktorteil 12, 14 zu einer Einheit zusammengefasst sein. Entsprechend ist die in Figur 1 gezeigte Konfiguration ausdrücklich nur als beispielhafte Ausführungsform zu verstehen.

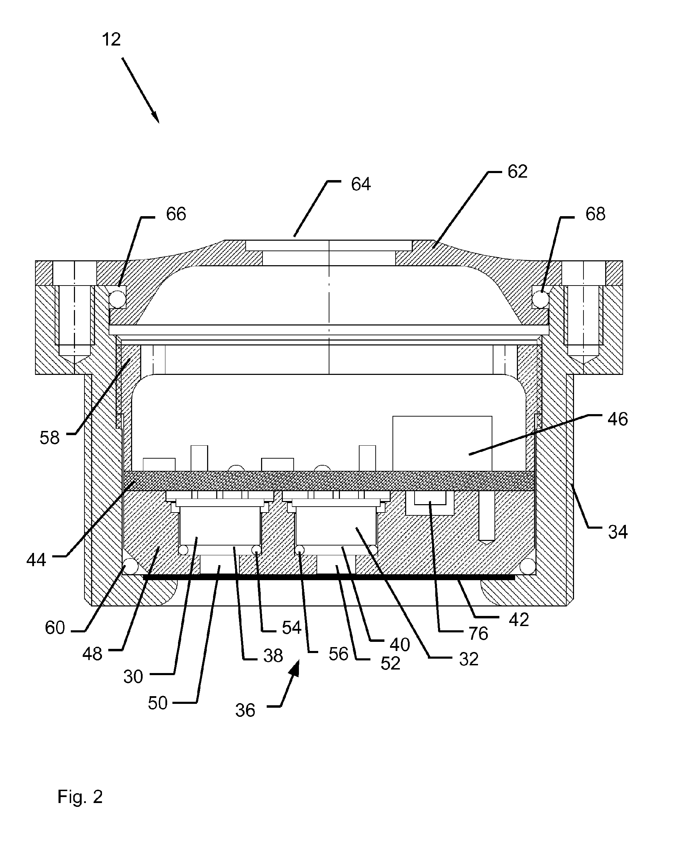

Die Darstellung in Figur 2 zeigt eine Schnittdarstellung einer Ausführungsform eines Sensorteils 12 der Flüssigkeitsrückhaltevorrichtung 10, wobei sich das Sensorteil 12 gegebenenfalls auch unabhängig von der Flüssigkeitsrückhaltevorrichtung 10 durch selbständige erfinderische Qualität auszeichnet.

Das Sensorteil 12 umfasst als Sensorik 30, 32 zumindest einen Glykolsensor, hier zwei Glykolsensoren, nämlich einen ersten Glykolsensor 30 und einen zweiten Glykolsensor 32. Das Sensorteil 12 umfasst die Sensorik 30, 32 in einem einseitig offenen Gehäuse 34 (Sensorgehäuse), wobei die Sensorik 30, 32 in dem Gehäuse 34 von einer Öffnung im Gehäuse 34 (Gehäuseöffnung 36) durch eine wasserundurchlässige, aber dampf- und/oder gasdiffusionsoffene Membran 38, 40 - im Folgenden kurz als Membran 38, 40 bezeichnet - zurückgesetzt ist. Bei der dargestellten Ausführungsform handelt es um jeweils eine jedem Glykolsensor 30, 32 individuell zugeordnete Membran 38, 40. Bei der dargestellten Ausführungsform wird die oder jede Membran 38, 40 mit einem Schutzgitter 42 in Form zum Beispiel eines Edelstahlmikrogewebes geschützt.

Die Sensorik 30, 32 ist auf einer der Oberflächen der Leiterplatte 44 (Platine) angebracht. Auf der anderen Oberfläche der Leiterplatte 44 befinden sich elektronische Bauteile zur Ansteuerung und Auswertung der Sensorik 30, 32, nämlich zumindest eine Verarbeitungseinheit 46 in Form von oder nach Art eines Mikroprozessors, ASICs oder dergleichen mit einem separaten Speicher oder einem in die Verarbeitungseinheit 46 integrierten Speicher. In dem Speicher ist ein im Betrieb des Sensorteils 12 durch die Verarbeitungseinheit 46 ausführbares Steuerungsprogramm gespeichert, das im Betrieb des Sensorteils 12 dessen Sensorik 30, 32, beim dargestellten Ausführungsbeispiel also den Betrieb der beiden Glykolsensoren 30, 32, überwacht. Im Speicher ist zumindest ein Grenzwert, optional ein Grenzwert und ein Schwellwert, gespeichert. Mittels der Verarbeitungseinheit 46 werden von der Sensorik 30, 32 gelieferte Messwerte mit dem Grenzwert oder dem Grenz- und dem Schwellwert verglichen. Im Falle einer Überschreitung des Grenzwerts ist mittels der Verarbeitungseinheit 46 ein vom Sensorteil 12 abgebbares und im Betrieb abgegebenes Sensorsignal 24 (Figur 1) zur Ansteuerung des Aktorteils 14 (Figur 1) generierbar.

Zu Aufnahme der Sensorik 30, 32 innerhalb des Gehäuses 34 ist ein Träger 48 vorgesehen. Dieser weist für jeden Glykolsensor 30, 32 eine Sensorkammer 50, 52 auf. Jede Sensorkammer 50, 52 ist zur formschlüssigen oder zumindest im wesentlichen formschlüssigen Aufnahme jeweils eines Glykolsensors 30, 32 vorgesehen und umfasst zwei Abschnitte, nämlich einen ersten Abschnitt zur Aufnahme des jeweiligen Glykolsensors 30, 32 und einen zweiten Abschnitt mit einem geringeren Durchmesser, in dem sich die Sensorkammer 50, 52 bis zur Unterseite des Trägers 48 und damit bis zur Gehäuseöffnung 36 fortsetzt. Im Übergang vom ersten Abschnitt der Sensorkammer 50, 52 zu deren zweitem Abschnitt befindet sich eine Dichtung in Form eines O-Rings 54, 56, die das Gehäuse des jeweiligen Glykolsensors 30, 32 zur Umgebung des Sensorteils 12 abdichtet.

Die Leiterplatte 44 liegt mit ihrer Unterseite auf dem Träger 48 auf. Innerhalb des Gehäuses 34 wird die Leiterplatte 44 mittels eines Druckorgans, bei dem es sich beim gezeigten Ausführungsbeispiel um einen Druckring 58 handelt, auf den Träger 48 gepresst. Die mit dem jeweiligen Druckorgan, hier also dem Druckring 58, ausgeübte Kraft setzt sich damit bis zum Träger 48 fort, der in das Gehäuse 34 und einen am Boden des Gehäuses 34 befindlichen O-Ring 60 gepresst wird. Gleichzeitig wird durch den Druck auf die Leiterplatte 44 der oder jeder Glykolsensor 30, 32 in dessen Sensorkammer 50, 52 auf den dortigen O-Ring 54, 56 gepresst. Damit ist auf Seiten der Gehäuseöffnung 36 das Innere des Gehäuses 34 gegen die Umgebung des Sensorteils 12 abgedichtet.

Der oder jeder Glykolsensor 30, 32 steht mit der Umgebung des Sensorteils 12 über den zwischen dem Schutzgitter 42 und der Membran 38, 40 offenen Teil der Sensorkammer 50, 52 in Kontakt. Dort eintretende Umgebungsluft oder dort gegebenenfalls eintretende Flüssigkeit kann auf die Anwesenheit von Glykol untersucht werden. Die Membran 38, 40 schützt dabei den jeweiligen Glykolsensor 30, 32 vor direktem Flüssigkeitskontakt. Die O-Ringe 54, 56, 60 gewährleisten die sichere Abdichtung des Innenraums des Sensorteils 12 gegen die Umgebungsluft und über die Gehäuseöffnung 36 eventuell eintretende Flüssigkeit.

In der Schnittdarstellung in Figur 2 ist nicht unmittelbar erkennbar, dass bei der hier beschriebenen Ausführungsform das Gehäuse 34 des Sensorteils 12 und auch der Innenraum des Gehäuses 34 rund und kreisförmig sind. Entsprechend ist auch der Druckring 58 kreisförmig und ist in ein im Innenraum des Gehäuses 34 gebildetes Gewinde einschraubbar. Beim Einschrauben des Druckrings 58 erfolgt das Anpressen der Leiterplatte 44 auf den Träger 48 und schließlich das Anpressen des Trägers 48 auf den O-Ring 60 sowie das Anpressen des oder jedes Glykolsensors 30, 32 auf den in der jeweiligen Sensorkammer 50, 52 befindlichen O-Ring 54, 56. Ein auf diese Weise im Inneren des Gehäuses 34 beweglicher Druckring 58 ist hinsichtlich des Aufbringens der zum Abdichten des Gehäuses 34 notwendigen Kraft besonders effizient, denn der Druckring 58 stützt sich mit seinem Außengewinde im Gehäuse 34 am dortigen Innengewinde ab. Das Eindrehen des Druckrings 58 in das Gewinde erlaubt darüber hinaus eine besonders feine Justierung der auf die Leiterplatte 44 wirkenden Kraft, so dass einerseits die notwendige Abdichtung des Gehäuses 34 erreicht ist und andererseits eine Beschädigung der Leiterplatte 44 vermieden wird. Schließlich ist die beim Eindrehen des Druckrings 58 auf die Leiterplatte 44 ausgeübte Kraft allseitig gleich, so dass unnötige mechanische Spannungen innerhalb der Leiterplatte 44 verhindert werden.

Das Gehäuse 34 ist auf der von der Gehäuseöffnung 36 abgewandten Seite durch einen Gehäusedeckel 62 verschlossen, der eine zentrale Öffnung aufweist, durch welche elektrische Versorgungs- und Datenleitungen zur Sensorik 30, 32 und/oder zur Verarbeitungseinheit 46 geführt werden können. Die eben genannte Öffnung kann als Stecker-Bohrung 64 ausgeführt sein, so dass ein ausreichend dichtender Stecker angebracht werden kann, der ein Hindurchführen der genannten Leitungen erlaubt. Der Gehäusedeckel 62 ist gegen das Gehäuse 34 abgedichtet und gezeigt ist insoweit eine O-Ringkammer 66, in welche als Dichtung ein passender O-Ring 68 eingesetzt wird. Der Gehäusedeckel 62 wird durch Verschrauben am Gehäuse 34 angebracht und in der Darstellung in Figur 2 sind insoweit entsprechende Schraubenlöcher sowie Gewindebohrungen im Gehäuse 34 gezeigt.

Das Sensorteil 12 weist durch die Abdichtung der bodenseitigen Gehäuseöffnung 36 und den sonstigen Abschluss des Gehäuses 34 mit dem Gehäusedeckel 62 eine hohe Schutzart auf und eignet sich damit für den Einsatz in rauer Umgebung, speziell zur Verwendung in einer Auffangwanne 16, wie dies in Figur 1 gezeigt ist.

Bei der Montage des Sensorteils 12 gemäß Figur 2 wird bei entferntem Gehäusedeckel 62 zunächst das grundsätzlich optionale Schutzgitter 42 in den Innenraum des Gehäuses 34 eingelegt. Dann folgt der bodenseitige O-Ring 60. Anschließend wird der Träger 48 und danach die Leiterplatte 44 mit der Sensorik 30, 32 in den Innenraum des Gehäuses 34 gegeben. Alternativ können der Träger 48 und die Leiterplatte 44 mit der Sensorik 30, 32 auch außerhalb des Gehäuses 34 kombiniert und kombinierten Zustand in den Innenraum des Gehäuses 34 gegeben werden. Jedenfalls findet sich danach der zur Abdichtung der jeweiligen Sensorkammer 50, 52 vorgesehene O-Ring 54, 56 in Kontakt mit dem Träger 48, zum Beispiel weil der O-Ring 54, 56 zuvor in die Sensorkammer 50, 52 eingelegt wurde oder sich bereits an einer Unterseite des jeweiligen Glykolsensors 30, 32 befindet. Schließlich wird der Druckring 58 in den Innenraum des Gehäuses 34 eingesetzt. Bei der hier gezeigten Ausführungsform wird der Druckring 58 in ein im Gehäuseinnenraum gebildetes Gewinde eingeschraubt und mit dem Einschrauben wird - wie oben erläutert - eine Kraft auf die Leiterplatte 44 ausgeübt, so dass einerseits der Träger 48 in den bodenseitigen O-Ring 60 und andererseits die Sensorik 30, 32 in die O-Ringe 54, 56 der Sensorkammern 30, 32 gepresst werden. Anschließend kann das Gehäuse 34 mittels des Gehäusedeckels 62 verschlossen werden.

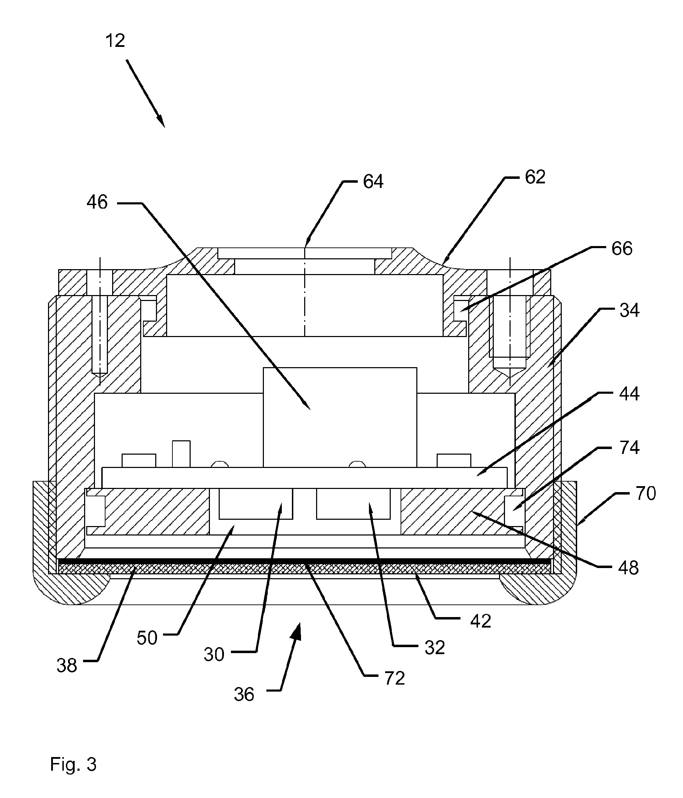

Die Darstellung in Figur 3 zeigt eine Schnittdarstellung einer alternativen Ausführungsform des Sensorteils 12 mit dem zumindest einen davon umfassten und als Sensorik 30, 32 fungierenden Glykolsensor 30, 32. Das Sensorteil 12 umfasst die Sensorik 30, 32 genau wie das in Figur 2 gezeigte Sensorteil 12 in einem einseitig offenen Gehäuse 34, wobei die Sensorik 30, 32 in dem Gehäuse 34 durch eine wasserundurchlässige, aber dampf- und/oder gasdiffusionsoffene Membran 38 der oben bereits beschriebenen Art von der Gehäuseöffnung 36 zurückgesetzt ist. Die Membran 38 ist mit einem Schutzgitter 42 geschützt.

Auch bei der in Figur 3 gezeigten Ausführungsform ist die Sensorik 30, 32 in einem Träger 48 platziert, der dafür eine die Sensorik 30, 32 aufnehmende und als Sensorkammer 50 fungierende Ausnehmung aufweist. Die Membran 38 liegt in der dargestellten Ausführungsform an einer Unterkante des Gehäuses 34 an und wird dort mittels eines als Gehäusekappe 70 fungierenden Gewindeklemmrings fixiert. Die dargestellte Ausführungsform kommt ohne eine separate Dichtung zum wasserdichten, aber dampfdiffusionsoffenen Verschließen des Gehäuses 34 aus, weil die Membran 38 auf ihrer zur Innenseite des Gehäuses 34 gewandten Oberfläche ein Beschichtung 72, zum Beispiel eine Beschichtung in Form von Teflon, aufweist, welche in Bezug auf das Verschließen des Gehäuses 34 als Dichtung fungiert. An die Stelle einer solchen Beschichtung kann alternativ allerdings durchaus eine randseitige Dichtung treten.

Nicht sichtbar ist in der Schnittdarstellung in Figur 3, dass es sich auch bei diesem Gehäuse 34 um ein kreisförmiges Gehäuse 34 handelt, das im Bereich der Gehäuseöffnung 36 ein Außengewinde aufweist, an dem die Gehäusekappe 70 anbringbar ist. Beim Anbringen der Gehäusekappe 70 am Gehäuse 34, hier also durch Verschrauben der Gehäusekappe 70 mit dem Gehäuse 34, werden die Membran 38 oder die Membran 38 und das grundsätzlich optionale Schutzgitter 42 im Gehäuse 34 fixiert. Dazu sind die Abmessungen der Membran 38 oder der Membran 38 und des Schutzgitters 42 auf einen Innendurchmesser der Gehäusekappe 70 abgestimmt, so dass die Membran 38 oder die Membran 38 und das Schutzgitter 42 in die Gehäusekappe 70 eingelegt werden können und beim Verschrauben der Gehäusekappe 70 mit dem Gehäuse 34 gegen eine allseitig umlaufende Unterkante des Gehäuses 34 gepresst werden.

Die Fixierung der Sensorik 30, 32 im Gehäuse 34 erfolgt beim in Figur 3 dargestellten Ausführungsbeispiel, indem der Träger 48 eine umlaufende O-Ringkammer 74 aufweist, in die vor dem Einsetzen des Trägers 48 in das Gehäuse 34 ein O-Ring (nicht gezeigt) eingelegt wird. Die Außenmaße des Trägers 48 sind in Relation zum Innendurchmesser des Gehäuseinnenraums (zumindest des relevanten Abschnitts des Gehäuseinnenraums) so bemessen, dass ein in die O-Ringkammer 74 eingelegter O-Ring mit Reibschluss an der Innenwand des betreffenden Gehäuseinnenraumabschnitts anliegt. Durch diesen Reibschluss sind der Träger 48 und damit auch die Sensorik 30, 32 im Gehäuse 34 fixiert. Alternativ kann die Fixierung des Trägers 48 im Gehäuse 34 auch im Zusammenhang mit dem Anbringen der Gehäusekappe 70 erfolgen. Dann wird der Träger 48 mittelbar - zum Beispiel mit der Membran 38 oder der Membran 38 und dem Schutzgitter 42 zwischen der Gehäusekappe 70 und dem Träger 48 - durch die Gehäusekappe 70 gegen ein im Gehäuseinnenraum gebildetes Profil gedrückt und so fixiert.

Bei der in Figur 3 gezeigten Ausführungsform des Sensorteils 12 sind die auch hier als Sensorik 30, 32 fungierenden Glykolsensoren 30, 32 auf einer Unterseite einer Leiterplatte 44 angebracht. Indem die Leiterplatte 44 mit den so angebrachten Glykolsensoren 30, 32 auf einer Oberseite des Trägers 48 angebracht ist, befindet sich die Sensorik 30, 32 in der im Träger 48 gebildeten Sensorkammer 50 und der oder jeder Glykolsensor 30, 32 weist in Richtung auf die Gehäuseöffnung 36 (und damit im angebrachten Zustand in Richtung auf die Wasseroberfläche in der Auffangwanne 16 oder im Abscheider 22).

Die Leiterplatte 44 umfasst neben der jeweiligen Sensorik 30, 32 weitere elektronische Bauteile, nämlich zumindest eine Verarbeitungseinheit 46 der oben bereits im Zusammenhang mit der Erläuterung der Ausführungsform in Figur 2 beschriebenen Art.

Bei einer Montage des Sensorteils 12 gemäß Figur 3 wird zunächst der Träger 48 mit der damit kombinierten Sensorik 30, 32 in den Innenraum des Gehäuses 34 gegeben. Anschließend wird die oben erwähnte Membran 38 in den Gehäuseinnenraum gegeben, so dass sich diese bei einer während der Montage nach oben gerichteten Gehäuseöffnung 36 oberhalb der Sensorik 30, 32 befindet. Gegebenenfalls wird im Anschluss an diese Membran 38 noch ein Schutzgitter 42 aufgelegt, das sich dann in gleicher Betrachtungsrichtung oberhalb der Membran 38 befindet. Mit dem Anbringen der Gehäusekappe 70 (Membran 38 oder Membran 38 und Schutzgitter 42 können sich dabei auch in der Gehäusekappe 70 und nicht in der Gehäuseöffnung 36 befinden) erfolgt eine Fixierung der Membran 38 oder der Membran 38 und des Schutzgitters 42 im Inneren des Gehäuses 34, so dass speziell die Membran so gelagert ist, das sie ihre Funktion zum Schutz der Sensorik 30, 32 vor direktem Wasserkontakt erfüllen kann.

Zur Fixierung der Membran 38 ist normalerweise eine in der Gehäusekappe 70 angebrachte Dichtung vorgesehen, die außen umlaufend am Rand der Membran 38 anliegt, oder eine Dichtung in Form eines Dichtrings, der zwischen Membran 38 und Schutzgitter 42 eingelegt wird und durch das Schutzgitter 42 von der Gehäusekappe 70 gegen die Membran 38 gepresst wird. Die Membran 38 wird damit wasserdicht an eine allseitig umlaufende Unterkante des Gehäuses 34 oder die Unterseite des Trägers 48 gepresst und in einer ebenen, ausgebreiteten Struktur gehalten, also quasi aufgespannt. Der Innenraum des Gehäuses 34 mit der dort befindlichen Sensorik 30, 32 ist auf diese Weise durch die Membran 38 oder die Membran 38 und die Dichtung wasserdicht abgeschlossen. Auf einer gegenüberliegenden Innenseite der Membran 38 ist optional ebenfalls eine solche Dichtung vorgesehen, die entweder im Inneren des Gehäuses 34 des Sensorteils 12 oder auf einer Unterseite des Trägers 48 angebracht ist. Bei zwei beidseitig der Membran 38 vorgesehenen Dichtungen wird mit diesen und mit der Membran 38 der Innenraum des Gehäuses 34 mit der dortigen Sensorik 30, 32 in besonders sicherer Art und Weise wasserdicht abgeschlossen und die Membran 38 wird ebenfalls zwischen den beiden Dichtungen in einer ebenen, ausgebreiteten Struktur gehalten, also aufgespannt. Gleiches lässt sich anstelle einer separaten Dichtung durch eine dichtende Beschichtung 72 der Membran 38 erreichen. Eine solche Beschichtung 72 der Membran 38 befindet sich auf deren im eingebauten Zustand zur Sensorik 30, 32 gewandten Oberfläche. Die Membran 38 wird dann mit der Gehäusekappe 70 gegen die Unterkante des Gehäuses 34 oder die Unterseite des Trägers 48 gepresst, so dass die Beschichtung 72 in ihrer Funktion als Dichtung wirksam wird. Zusätzlich oder alternativ kann sich eine solche Beschichtung 72 auch auf der anderen Oberfläche der Membran 38 befinden. Die Beschichtung 72 muss nicht notwendig vollflächig aufgebracht sein und kann sich demnach auch auf den Randbereich der Membran 38 beschränken.

Das Sensorteil 12 gemäß Figur 2 oder gemäß Figur 3 kann in nicht dargestellter Art und Weise, zum Beispiel als Bestandteil des oder jedes Glykolsensors 30, 32, eine Heizeinrichtung umfassen, so dass für den Erhalt von Messwerten von der Sensorik 30, 32 von definierten Temperaturen ausgegangen werden kann. Darüber hinaus oder alternativ kann das Sensorteil 12 gemäß Figur 2 oder gemäß Figur 3 ebenfalls in nicht dargestellter Art und Weise im Bereich der Sensorik 30, 32, insbesondere auf derselben Seite der Membran 38, einen Feuchtigkeitssensor zur Überwachung der Luftfeuchtigkeit der über die Gehäuseöffnung 36 in das Innere des Sensorteils 12 eintretenden Umgebungsluft aufweisen. Als Ort für die Anbringung eines solchen Feuchtigkeitssensors kommt die Sensorkammer 50 oder eine ähnliche, eigene Ausnehmung in Betracht, so dass der Feuchtigkeitssensor bei einer üblichen Anbringung des Sensorteils 12 genau wie die Sensorik 30, 32 nach unten gewandt und damit auf eine Wasserfläche oder dergleichen unterhalb des Sensorteils 12 gerichtet ist.

Das Sensorteil 12 gemäß Figur 2 oder gemäß Figur 3 ist nicht auf die erwähnte kreisrunde Grundform beschränkt. Genauso kommt eine polygonale, zum Beispiel viereckige Grundform in Betracht. Der O-Ring 60 (Fig. 2) ist dann idealerweise eine Dichtung mit einer entsprechenden Form. Der in die O-Ringkammer 74 (Fig. 3) einzulegende O-Ring passt sich aufgrund seiner Elastizität an die jeweilige Randkontur des Trägers 48 an. Die Gehäusekappe 70 (Fig. 3) kann auch als zum Beispiel viereckige Gehäusekappe 70 auf das Gehäuse 34 gesetzt und im aufgesetzten Zustand zum Beispiel außen verschraubt werden. Der Druckring 58 (Fig. 2) kann ebenfalls als zum Beispiel viereckiger Druckring 58 in das Innere eines entsprechend geformten Gehäuses 34 eingesetzt und mit entlang der Umfangslinie des Druckrings 58 verteilten Schrauben oder dergleichen auf die Leiterplatte 44 gepresst werden. Darüber hinaus kommt auch bei einem außen viereckigen Gehäuse 34 durchaus ein kreisrunder Innenraum im Gehäuse 34 in Betracht, so dass dort ein runder Druckring 58 in ein entsprechendes Gewinde eingeschraubt werden könnte. Die Erwähnung der kreisförmigen Grundform ist demnach ausdrücklich nur als Beschreibung einer möglichen Ausführungsform zu verstehen. Die Kreisform erlaubt allerdings eine leichte und vor allem genaue Fertigung der Einzelteile und später eine besonders einfache Montage, zum Beispiel indem einzelne Teile (Druckring 58 und Gehäuse 34; Gehäusekappe 70 und Gehäuse 34) mittels eines Gewindes und durch einfaches Verschrauben verbunden werden.

Bei der in Figur 2 gezeigten Ausführungsform des Sensorteils 12 ist noch ein Leuchtelement 76, zum Beispiel eine LED oder dergleichen, vorgesehen, welches die Betriebsbereitschaft des Sensorteils 12 und gegebenenfalls auch eine eventuelle Fehlersituation des Sensorteils 12 anzeigt. Dafür ist das Leuchtelement 76 an der Unterseite der Leiterplatte 44 angebracht und strahlt damit in Richtung auf die Gehäuseöffnung 36. Der mittels des Leuchtelements 76 angezeigte Status des Sensorteils 12 kann so also leicht und ohne dass dafür ein Öffnen des Sensorteils 12 notwendig wäre, geprüft werden. Zur Aufnahme des Leuchtelements 76 ist im Träger 48 eine entsprechende Ausnehmung vorgesehen, die einerseits nicht bis zur Unterkante des Trägers 48 reicht und andererseits aber, zum Beispiel aufgrund des Materials, aus dem der Träger 48 gefertigt ist oder aufgrund einer am Boden der Ausnehmung bis zur Unterkante des Trägers 48 noch verbleibenden Materialstärke, eine Erkennbarkeit der vom Leuchtelement 76 ausgesandten optischen Signale erlaubt.

Der oder jeder Glykolsensor 30, 32 ist bei einer besonderen Ausführungsform mit seinen Kontaktstiften in in der Leiterplatte 44 gebildete Kontaktöffnungen eingesetzt, so dass der Glykolsensor 30, 32 mittels einer lösbaren Steckverbindung und nicht durch eine Lötverbindung oder dergleichen elektrisch leitend mit den auf der Leitplatte 44 befindlichen Leiterbahnen verbunden ist. Auf diese Weise kann ein Glykolsensor 30, 32 im Fehlerfall oder zu Wartungszwecken leicht und unkompliziert gegen einen anderen Glykolsensor 30, 32 ausgetauscht werden.

Einzelne im Vordergrund stehende Aspekte der hier eingereichten Beschreibung lassen sich damit kurz wie folgt zusammenfassen: Angegeben werden eine Flüssigkeitsrückhaltevorrichtung 10 mit einem Sensorteil 12 und einem mittels des Sensorteils 12 ansteuerbaren Aktorteil 14, sowie ein derartiges Sensorteil 12 als Einzelelement, wobei das Sensorteil 12 eine in Form zumindest eines Glykolsensors 30, 32 realisierte Sensorik 30, 32 in einem einseitig offenen Gehäuse 34 umfasst und wobei die Sensorik 30, 32 in dem Gehäuse 34 durch eine wasserundurchlässige, aber dampfdiffusionsoffene Membran 38, 40 von der Gehäuseöffnung 36 zurückgesetzt ist.

### Bezugszeichenliste

- 10: Flüssigkeitsrückhaltevorrichtung
- 12: Sensorteil
- 14: Aktorteil
- 16: Auffangwanne
- 18: Klimaanlage / leckagegefährdetes Aggregat
- 20: Abfluss
- 22: Abscheider
- 24: Sensorsignal
- 26, 28: (frei)
- 30: (erster) Glykolsensor
- 32: (zweiter) Glykolsensor
- 34: Gehäuse
- 36: Gehäuseöffnung
- 38, 40: Membran
- 42: Schutzgitter
- 44: Leiterplatte
- 46: Verarbeitungseinheit
- 48: Träger
- 50, 52: Sensorkammer
- 54, 56: O-Ring
- 58: Druckring
- 60: O-Ring
- 62: Gehäusedeckel
- 64: Stecker-Bohrung
- 66: O-Ringkammer
- 68: O-Ring
- 70: Gehäusekappe
- 72: Beschichtung
- 74: O-Ringkammer
- 76: Leuchtelement

## Patentansprüche

1. Flüssigkeitsrückhaltevorrichtung (10) mit einem Sensorteil (12) und einem Aktorteil (14) zur Verwendung mit einer Auffangwanne (16) unter einem leckagegefährdeten Aggregat (18) mit einem darin befindlichen wassergefährdenden, flüssigen und zurückzuhaltendem Medium,
wobei mittels einer von dem Sensorteil (12) umfassten Sensorik (30, 32) das wassergefährdende Medium in der Auffangwanne (16) sensierbar und mittels des Sensorteils (12) gegebenenfalls ein diesbezügliches Sensorsignal (24) generierbar ist,
wobei mittels des Sensorsignals (24) das Aktorteil (14) ansteuerbar ist, wobei das Aktorteil (14) als Sicherheitseinrichtung fungiert oder eine Sicherheitseinrichtung umfasst, mittels derer ein Austritt des wassergefährdenden flüssigen Mediums aus der Auffangwanne (16) verhinderbar ist und
wobei als Sensorik (30, 32) des Sensorteils (12) zumindest ein Glykolsensor (30, 32) fungiert.

2. Flüssigkeitsrückhaltevorrichtung (10) nach Anspruch 1, mit zumindest zwei in dem Sensorteil (12) als Sensorik (30, 32) fungierenden Glykolsensoren (30, 32), insbesondere zumindest zwei redundant betriebenen Glykolsensoren (30, 32).

3. Flüssigkeitsrückhaltevorrichtung (10) nach einem der Ansprüche 1 oder 2, mit einem Sensorteil (12) mit einem aufgrund einer Gehäuseöffnung (36) einseitig offenen Gehäuse (34), wobei die Sensorik (30, 32) in dem Gehäuse (34) durch eine wasserundurchlässige, aber dampfdiffusionsoffene Membran (38, 40) von der Gehäuseöffnung (36) zurückgesetzt ist.

4. Flüssigkeitsrückhaltevorrichtung (10) nach Anspruch 3,
wobei das Gehäuse des Sensorteils (12) kreisförmig ist und in einen Innenraum des Gehäuses (34) ein ebenfalls kreisförmiger Träger (48) sowie eine von dem Träger (48) gehaltene, ebenfalls kreisförmige Leiterplatte (44), welche die Sensorik (30, 32) umfasst, einsetzbar ist,
wobei der Träger (48) zur Aufnahme des oder jedes als Sensorik (30, 32) fungierenden Glykolsensors (30, 32) jeweils eine Sensorkammer (50, 52) aufweist,
wobei der Träger (48) zum Gehäuse (34) und der oder jeder Glykolsensor (30, 32) zur Sensorkammer (50, 52) mit einer Dichtung, insbesondere einer Dichtung in Form eines O-Rings (60, 54, 56), abgedichtet ist und
wobei in den Innenraum des Gehäuses (34) ein Druckring (58) einsetzbar ist, der im angebrachten Zustand auf die Leiterplatte (44) und zumindest mittelbar auf den Träger (48) wirkt und einen dichtenden Kontakt zwischen dem Träger (48), dem Innenraum des Gehäuses (34) und dem dort befindlichen O-Ring (60) einerseits sowie zwischen dem oder jedem Glykolsensor (30, 32), der jeweiligen Sensorkammer (50, 52) und dem dort jeweils befindlichen O-Ring (54, 56) herstellt.

5. Flüssigkeitsrückhaltevorrichtung (10) nach Anspruch 4, wobei der Druckring (58) im Innenraum des Gehäuses (34) in ein dort befindliches Gewinde einschraubbar ist.

6. Flüssigkeitsrückhaltevorrichtung (10) nach Anspruch 3, mit einem Sensorteil (12) mit einem kreisförmigen Gehäuse (34), das im Bereich der Gehäuseöffnung (36) ein Außengewinde aufweist, an dem eine Gehäusekappe (70) anbringbar ist, wobei die Gehäusekappe (70) die Sensorik (30, 32) und die Membran (38) im Gehäuse (34) fixiert.

7. Flüssigkeitsrückhaltevorrichtung (10) nach einem der vorangehenden Ansprüche, mit einem Sensorteil (12) mit einer Heizeinrichtung im Bereich der Sensorik (30, 32), insbesondere auf derselben Seite der Membran (38) wie die Sensorik (30, 32).

8. Flüssigkeitsrückhaltevorrichtung (10) nach einem der vorangehenden Ansprüche, mit einem Sensorteil (12) mit einer Verarbeitungseinheit (46) und einem Speicher, wobei in dem Speicher ein durch die Verarbeitungseinheit (46) ausführbares Steuerungsprogramm gespeichert ist, das im Betrieb des Sensorteils (12) dessen Sensorik (30, 32) überwacht, wobei im Speicher ein durch die Verarbeitungseinheit (46) mit einem der Sensorik (30, 32) gelieferten Messwert vergleichbarer Grenzwert gespeichert ist und wobei durch die Verarbeitungseinheit (46) im Falle einer Überschreitung des Grenzwerts durch den Messwert ein Sensorsignal (24) generierbar und mittels des Sensorsignals (24) der Aktorteil (14) der Flüssigkeitsrückhaltevorrichtung (10) ansteuerbar ist.

9. Flüssigkeitsrückhaltevorrichtung (10) nach Anspruch 8, mit einem Sensorteil (12) mit einer Busschnittstelle und einer über einen an die Busschnittstelle angeschlossenen Bus parametrierbaren Verarbeitungseinheit (46) sowie einem über den an die Busschnittstelle angeschlossenen Bus übermittelbaren Sensorsignal (24).

10. Sensorteil (12) einer Flüssigkeitsrückhaltevorrichtung (10) nach einem der vorangehenden Ansprüche,
wobei das Sensorteil (12) und die Flüssigkeitsrückhaltevorrichtung (10) zur Verwendung mit einer Auffangwanne (16) unter einem leckagegefährdeten Aggregat (18) mit einem darin befindlichen wassergefährdenden, flüssigen und zurückzuhaltendem Medium bestimmt und eingerichtet sind,
wobei mittels des Sensorteils (12) das wassergefährdende Medium in der Auffangwanne (16) sensierbar und gegebenenfalls ein diesbezügliches Sensorsignal (24) generierbar ist.
